# EUROPEAN PATENT APPLICATION

(11) **EP 4 667 582 A1**
(43) Date of publication of application: **24.12.2025**
(21) Application number: 25182932.1
(22) Date of filing: 16.06.2025
(51) Int. Cl.: C12Q 1/56, A61K 38/48, G01N 33/86

(54) **FACTOR XA REAGENT**

(30) Priority: 20.06.2024 US 202418748840; 12.09.2024 LU 508259
(71) Applicant: Instrumentation Laboratory Company, Bedford, MA 01730 (US)
(72) Inventor: CAO, Zhenghua, Bedford 01730 (US); KUNG, Chun, Bedford 01730 (US)
(74) Representative: Müller-Boré & Partner Patentanwälte PartG mbB

(57) **Abstract**

An example composition includes factor Xa, a substrate, one or more of guanidine derivatives, and, in some cases, heparin. An example method for detecting an anticoagulant in a sample includes combining or contacting the sample with the composition and measuring activity of the factor Xa based on a state of the substrate. The composition modulates the activity of the factor Xa in the sample, where modulation includes reducing a rate of coagulation of the sample caused by factor Xa. A presence of the anticoagulant is based on the activity of the factor Xa.

## Description

This specification relates generally to example factor Xa reagent compositions and uses thereof.

Factor Xa (FXa) is the activated form of coagulation factor X, or thrombokinase. Factor X is an enzyme that promotes coagulation of a test sample, such as whole blood or plasma. An example factor Xa inhibitor is an anticoagulant that functions by selectively blocking activity of factor Xa, thereby preventing or inhibiting clot formation in the test sample.

An example composition includes factor Xa, a substrate, and one or more of guanidine derivatives. The composition may include one or more of the following features, either alone or in combination.

The composition may include heparin. The one or more guanidine derivatives may be or include 1-methylguanidine, 1,1-dimethylguanidine, 1,1-diethylguanidine, or N-benzyl-N-methylguanidine, where N indicates a substituent on nitrogen. The one or more guanidine derivatives may be or include a mono-alkylated guanidine derivative. The one or more guanidine derivatives may be or include a dialkylated guanidine derivative. The one or more guanidine derivatives may be or include alkylated guanidine having a chemical structure represented by formula: 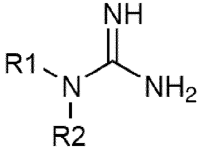 where R1 is H, methyl, ethyl, or benzyl, where R2 is H, methyl, ethyl, or benzyl, and where N is nitrogen and H is hydrogen. The one or more guanidine derivatives may include a mono-alkylated guanidine including an alkyl group having a length that is different from a length of an alkyl group of 1-methylguanidine, or a dialkylated guanidine including an alkyl group having a length that is different from a length of an alkyl group of 1,1-dimethylguanidine or 1,1-diethylguanidine. The one or more guanidine derivatives may be or include a sulfate salt. The substrate may be or include a chromogenic substrate. The composition may be included in an apparatus that is, or includes, a kit or that is, or includes, a cartridge.

An example method for detecting an anticoagulant in a sample includes combining or contacting the sample with a composition including factor Xa, a guanidine derivative, and a substrate, and measuring activity of the factor Xa based on a state of the substrate, where a presence of the anticoagulant is based on the activity of the factor Xa. The method may include one or more of the following features, either alone or in combination.

The anticoagulant may be or include a factor Xa inhibitor, which may be a direct factor Xa inhibitor. The anticoagulant may be or include a factor Xa inhibitor including one or more of rivaroxaban, apixaban, edoxaban, or betrixaban. The anticoagulant may be or include a factor Xa inhibitor including an indirect factor Xa inhibitor based on antithrombin. The indirect factor Xa inhibitor may be or include one or more of low molecular weight heparin, unfractionated heparin, fondaparinux, or danaparoid. The substrate may be or include a color change in the substrate. The composition may modulate the activity of the factor Xa in the sample, where modulation comprises reducing a rate of coagulation or substrate cleavage caused by factor Xa.

Any two or more of the features described in this specification, including in this summary section, can be combined to form implementations not specifically described herein.

The systems and processes described herein, or portions thereof, may be implemented as one or more apparatus or as a method and may include one or more processing devices and computer memory to store executable instructions to implement control of various functions. The systems, processes, and compositions, including but not limited to apparatus, methods, and/or reagents, described herein may be configured, for example, through design, manufacture, construction, combination of two or more substances, arrangement, placement, programming, operation, activation, deactivation, and/or control.

The details of one or more implementations are set forth in the accompanying drawings and the description below. Other features, objects, and advantages will be apparent from the description and drawings, and from the claims.

The figures show:
Figs. 1A to 1G show structural formulas of examples of guanidine derivatives that may be used in the example factor Xa reagents described herein.
Fig. 2A is a flowchart showing an example process that may be performed to detect factor Xa inhibitors in a test sample using a chromogenic assay.
Fig. 2B is a flowchart showing an example process that may be performed to detect factor Xa inhibitors in a test sample using a fluorogenic assay.
Fig. 3 is a graph containing example plots showing factor Xa inhibition on the Y axis for different concentrations of salt or guanidine derivatives on the X axis.
Fig. 4 is a graph containing example plots showing factor Xa inhibition on the Y axis for different concentrations of guanidine derivatives on the X axis.
Fig. 5 is an example plot showing residual factor Xa activity on the Y axis for different concentrations of direct factor Xa inhibitors on the X axis.
Fig. 6 is a graph containing example plots showing substrate turnover rates on the Y axis for different levels of factor Xa activity on the X axis for both dimethylguanidine and salt.
Fig. 7 is a graph containing example plots showing chromogenic activity on the Y axis for different reaction rates on the X axis for reactions containing different amounts of dimethylguanidine.
Fig. 8 is an example plot showing a linear correlation of factor Xa activity on the Y axis with unfractionated heparin concentrations on the X axis in a reaction mixture containing dimethylguanidine.
Fig. 9 is an example plot showing factor Xa activity on the Y axis as compared to antithrombin activity on the X axis absent dimethylguanidine.
Fig. 10 is a graph containing example plots showing, for different factor Xa concentrations, factor Xa activity as compared to antithrombin activity in the presence of 20 millimoles (mM) of dimethylguanidine.

Like reference numerals in different figures indicate like elements.

Described herein are examples of reagents, which are referred to herein as "factor Xa reagents", that modulate the activity of factor Xa in a reaction in an assay. Modulation in this context may include affecting a rate of coagulation of a test sample caused by factor Xa. For example, modulating may include reducing a rate of coagulation of the test sample caused by factor Xa relative to rate of coagulation of the test sample that would have occurred absent the presence of the factor Xa reagent. Modulating factor Xa activity may have advantages, such as allowing for the optimization of reaction kinetics and sample-to-reagent volume ratios in an assay that detects coagulation of a test sample caused by factor Xa, such that greater test sample volumes and greater amounts and/or concentrations of factor Xa can be used in the assay relative to what would have been used in the same assay absent the factor Xa reagent. Greater test sample volumes and greater amounts and/or concentrations of factor Xa may facilitate detection of factor Xa inhibitors or reversal of such inhibitors in the assay. Slower reaction rates may enable more accurate detection of factor Xa inhibitors or reversal of such inhibitors in the assay.

The factor Xa reagents may be in solid form such as a powder or film. In some implementations, the factor Xa reagent may be lyophilized or dried. The factor Xa reagents may be in liquid form, or in a solid form, over a temperature range of at least 0° Celsius (C) to 100° C. All temperatures described herein are at a standard pressure of 1 atmosphere (101.325 kilopascal).

The test sample may be whole blood, a part of whole blood, a sample derived from whole blood, a part of whole blood that has been depleted of one or more blood constituents (e.g., depleted of leukocytes, erythrocytes, platelets, or protein (e.g., albumin)), or a constituent of whole blood that has been isolated. In an example, the test sample may be platelet-free plasma, which is whole blood that has been depleted of a blood constituent (platelets). The test sample may be collected from a subject, such as a human, using standard techniques.

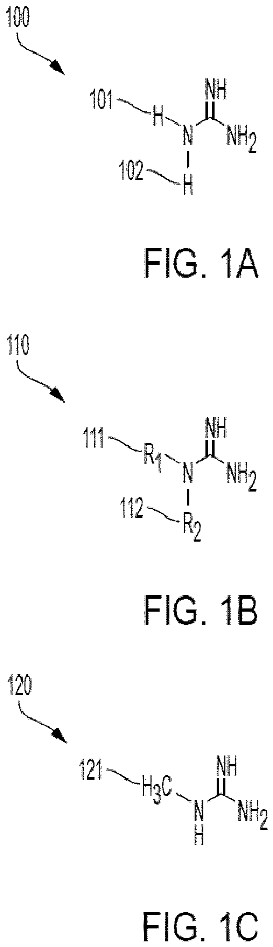

An example factor Xa reagent may include factor Xa, a substrate, and one or more constituents that modulate the activity of the factor Xa in a test sample. An aqueous buffer may be included in some implementations. Examples of the one or more constituents that may be contained in the factor Xa reagent to modulate factor Xa activity in a test sample include guanidine or one or more guanidine derivatives. Guanidine is a nitrogen-rich organic compound having the formula HNC(NH₂)₂. The structural formula of guanidine 100 is shown in Fig. 1A.

An example of a guanidine derivative that may be contained in the factor Xa reagent to modulate factor Xa activity may be an alkylated guanidine. Guanidine derivatives, such as alkylated guanidines and others described herein, modulate factor Xa amidolytic activity. Amidolytic activity refers to cleavage of a peptide bond in a polypeptide or protein by a protease enzyme, including factor Xa. The factor Xa activity described herein includes amidolytic activity unless stated otherwise.

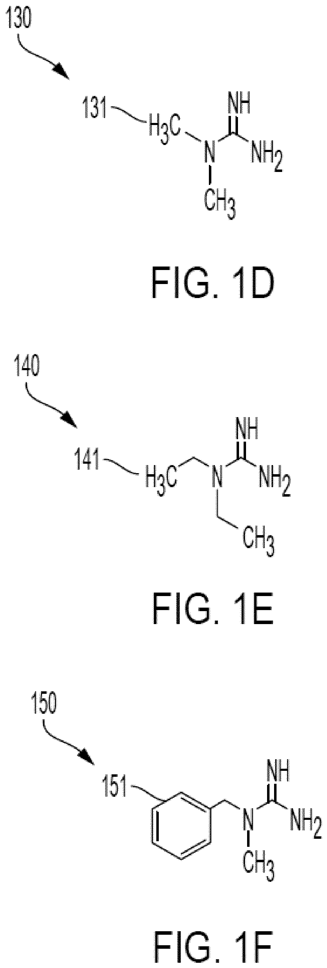

An alkylated guanidine may have one or both of its hydrogen (H) atoms 101 and 102 replaced with a larger chemical group, represented as R1 111 and R2 112 in Fig. 1B, where R1 111 may be a methyl group 131 (Fig. 1D), an ethyl group 141 (Fig. 1E), or a benzyl group 151 (Fig. 1F); and R2 112 may be a methyl group, an ethyl group, or a benzyl group. A methyl group 131 includes an alkyl derived from methane, containing one carbon atom bonded to three hydrogen atoms, having the chemical formula -CH₃. An ethyl group 141 includes an alkyl substituent with the formula -CH₂CH₃ derived from ethane (C₂H₆). A benzyl group 151 includes a substituent or molecular fragment having the structure R-CH₂-C₆H₅. Benzyl features a phenyl group (C₆H₅) attached to a methylene group (-CH₂-) group.

Other examples of guanidine derivatives that may be included in the factor Xa reagent to modulate factor Xa activity may include a mono-alkylated guanidine, a dialkylated guanidine, or a combination of a mono-alkylated guanidine and a dialkylated guanidine. Non-limiting examples of guanidine derivatives that may be included in the factor Xa reagent may include: 1-methylguanidine, 1,1-dimethylguanidine, 1,1-diethylguanidine (C₅H₁₃N₃), or N-benzyl-N-methylguanidine, where N indicates a substituent on nitrogen. Fig. 1C shows the structural formula for 1-methylguanidine 120, Fig. 1D shows the structural formula for 1,1-dimethylguanidine 130, Fig. 1E shows the structural formula for 1,1-diethylguanidine 140, and Fig. 1F shows the structural formula for N-benzyl-N-methylguanidine 150.

In an example, methylguanidine, which is a methylated guanidinium, C₂H₈N₃⁺, under physiological pH, modulates factor Xa activity. The rate of modulation is enhanced by methylation at the second 1N position (1,1-dimethylated guanidine, C₃H₁₀N₃⁺). This rate can be adjusted by the length of the alkyl groups, or by adding a bulky group, such as an aromatic ring, to the second 1N position.

Other examples of guanidine derivatives that may be included in the factor Xa reagent to modulate factor Xa activity may include a mono-alkylated guanidine derivative that contains an alkyl group that is different in length than the alkyl group in 1-methylguanidine 120 of Fig. 1C. For example, an alkyl group that is different in length than the alkyl group in 1-methylguanidine 120 may include two or more carbon atoms. An alkyl group with two carbon atoms has the formula -CH₂CH₃. A mono-alkylated guanidine derivative that contains an alkyl group with two carbon atoms has the formula C₃H₉N₃. An alkyl group with three carbon atoms has the formula -CH₂CH₂CH₃. A mono-alkylated guanidine derivative that contains an alkyl group with three carbon atoms has the formula C₄H₁₁N₃. An alkyl group with four carbon atoms has the formula -CH₂CH₂CH₂CH₃. A mono-alkylated guanidine derivative that contains an alkyl group with four carbon atoms has the formula C₅H₁₃N₃.

Other examples of guanidine derivatives that may be included in the factor Xa reagent to modulate factor Xa activity may include dialkylated guanidine derivatives that contains one or both alkyl groups that are different in length than the alkyl groups in 1,1-dimethylguanidine 130 of Fig. 1D. Alkyl groups that are different in length than the alkyl groups in 1,1-dimethylguanidine 130 may include one or more carbon atoms. An alkyl group with one carbon has the formula -CH₃. A dialkylated guanidine derivative that contains one alkyl group with one carbon and one alkyl group with two carbon atoms has the formula C₄H₁₁N₃.

Other examples of guanidine derivatives that may be included in the factor Xa reagent to modulate factor Xa activity may include dialkylated guanidine derivatives that contain one or both alkyl groups that are different in length than 1,1-diethylguanidine 140 of Fig. 1E. Alkyl groups that are different in length than the alkyl groups in 1,1-diethylguanidine 140 may include three or more carbon atoms. An alkyl group with three carbon atoms has the formula -CH₂CH₂CH₃. A dialkylated guanidine derivative that contains a one alkyl group with two carbon atoms and one alkyl group with three carbon atoms has the formula C₆H₁₅N₃. An alkyl group with four carbon atoms has the formula -CH₂CH₂CH₂CH₃. A dialkylated guanidine derivative that contains one alkyl group with two carbon and one alkyl group with four carbon atoms has the formula C₇H₁₇N₃.

Other examples of guanidine derivatives that may be included in the factor Xa reagent to modulate factor Xa activity may include a dialkylated guanidine derivative that contains one alkyl group that is longer in length than the alkyl groups in 1,1-dimethylguanidine 130 or 1,1-diethylguanidine 140 and has another alkyl group that is the same length or shorter than the alkyl groups in 1,1-dimethylguanidine 130 or 1,1-diethylguanidine 140. An alkyl group with the same length as the alkyl groups in 1,1-dimethylguaninde 130 has the chemical formula -CH₃. An alkyl group with the same length as the alkyl groups in 1,1-diethylguaninde 140 has the chemical formula -CH₂CH₃.

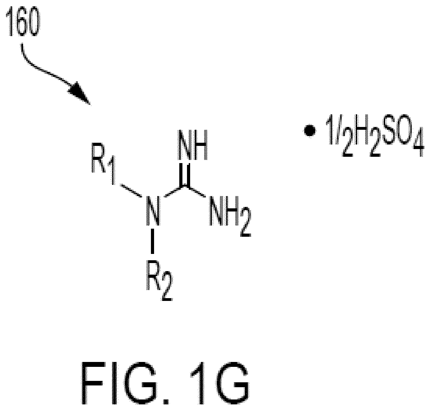

Other examples of guanidine derivatives that may be included in the factor Xa reagent to modulate factor Xa activity may include a salt containing a conjugate acid of guanidine, which has the chemical formula C(NH₂)₃⁺. Examples of guanidine-derivative salts may include a conjugate acid of guanidine, a carbonate salt containing a conjugate acid of guanidine, a chloride salt containing a conjugate acid of guanidine, a nitrate salt containing a conjugate acid of guanidine, a perchlorate salt containing a conjugate acid of guanidine, and/or a picrate salt containing a conjugate acid of guanidine. An example of guanidine-derivative salts that may be included in the factor Xa reagent may include guanidine in a sulfate salt 160 having the structural formula shown in Fig. 1G.

The guanidine derivatives that may be included in the factor Xa reagent to modulate factor Xa activity may include a combination of two or more of the example guanidine derivatives described herein. Example combinations of guanidine derivates that may be included in the factor Xa regent to modulate factor Xa activity may be a combination of 1-methylguanidine 120 and 1,1-dimethylguanidine 130, a combination of 1-methylguanidine 120 and 1,1-diethylguanidine 140, a combination of 1-methylguanidine 120 and N-benzyl-N-methylguanidine 150, a combination of 1,1-dimethylguanidine 130 and 1,1-diethylguanidine 140, a combination of 1,1-diethylguanidine 140 and N-benzyl-N-methylguanidine 150, and/or a combination of 1,1-dimethylguanidine 130 and N-benzyl-N-methylguanidine 150.

The guanidine derivatives that may be included in the factor Xa reagent to modulate factor Xa activity may include a combination of one or more of the example guanidine derivatives described herein and guanidine itself (Fig. 1A). Example combinations of guanidine and guanidine derivatives that may be included in the factor Xa regent to modulate factor Xa activity may be a combination of guanidine and 1-methylguanidine, a combination of guanidine and 1,1-dimethylguanidine, a combination of guanidine and 1,1-diethylguanidine, and/or a combination of guanidine and N-benzyl-N-methylguanidine.

In some implementations, guanidine and/or individual guanidine derivatives described herein may each be present in the factor Xa reagent at a concentration of about 0.1 to 100 millimoles (mM) guanidine derivative for every one nkat/mL of factor Xa. One katal (kat) is the amount of enzyme that converts one mole of substrate per second. Here, "nkat" refers to nanokatals. "About" as used herein allows for a deviation from a given number, such as, but not limited to, a deviation of 1%, 2%, 3%, 4%, or 5%.

In some implementations an aqueous buffer that may be included in a liquid version of the factor Xa reagent may be or include 2-morpholin-4-ylethanesulfonic acid (MES) or 3-(N-morpholino) propanesulfonic acid (MOPS), which is a structural analog to MES. In some implementations an aqueous buffer in the factor Xa reagent may be or include HEPES ((4-(2-hydroxyethyl)-1-piperazineethanesulfonic acid), Bis-Tris buffer, citrate, ADA (N-(2-acetamido)iminodiacetic acid, N-(carbamoylmethyl)iminodiacetic acid) buffer, ACES (N-(2-Acetamido)-2-aminoethanesulfonic acid) buffer, PIPES (piperazine-N,N'-bis(2-ethanesulfonic acid)) buffer, imidazole/imidazolium buffer, Bis-Tris Propane buffer, maleic acid buffer, phosphate buffer, MOPSO (2-Hydroxy-3-morpholinopropanesulfonic acid) buffer, BES (bis(2-hydroxyethyl)-2-amino-ethansulfonic acid) buffer, MOPS buffer, TES (tris(hydroxymethyl)methyl-2-aminomethane sulfonic acid) buffer, and/or MOPS (3-(N-morpholino)propanesulfonic acid) buffer.

In some implementations an aqueous buffer in the factor Xa reagent may be or include one or more carboxylic acid derivatives. Examples of carboxylic acid derivatives that may be included in the aqueous buffer may include the carboxylic acid salt acetates, ethylene diamine tetra acetate, butane tetra carboxylate, propane tricarboxylate, citrate, succinate, tartarate, malonate, and/or gluconate.

In some implementations an aqueous buffer in the factor Xa reagent may include a preservative. Examples of preservatives that may be included in the aqueous buffer may include ProClin^{™} 300 (3% 5-chloro-2-methyl-4-isothiazolin-3-one (CMIT) and 2-methyl-4-isothiazloin-3-one (MIT)), sodium azide, gentamicin, thimerosal, butylated hydroxytoluene (BHT), sucrose, trehalose, glycerin, sodium citrate, poloxamer, cetyl trimethyl ammonium bromide (CTAB), or a combination of two or more of these.

In some implementations one or more substrates may be included in the factor Xa reagent to measure the amount of factor Xa activity in a test sample in the presence of a factor Xa inhibitor. Examples of substrates that may be included in the factor Xa reagent include a chromogenic substrate. In some examples, a chromogenic substrate is a colorless substance that transforms into a colored substance in response to an enzymatic reaction. In an example, a chromogenic substrate changes its state, or color, based on the amount of factor Xa activity in a test sample. Thus, the chromogenic substrate enables measuring activity of the factor Xa based on the color of the substrate, as described below. Examples of chromogenic substates that may be used in the factor Xa reagent include, but are not limited to, (S)-2-((2S,3S)-2-(2,5-dioxopyrrolidin-1-yl)-3-methylpentanamido)-N-(2-(((S)-5-guanidino-1-((4-nitrophenyl)amino)-1-oxopentan-2-yl)amino)-2-oxoethyl)-5-oxo-5-(piperidin-1-yl)pentanamide hydrochloride (Pefachrome^{®} factor Xa S-2732^{™}), S-2222^{™} (Bz-Ile-Glu( -OR)-Gly-Arg-pNA•HCl), S-2765^{™} (Z-D-Arg-Gly-Arg-pNA•2HCl), and SPECTROZYME FXa (CH3O-CO-D-CHG-Gly-ArgpNA.AcOH).

Other examples of substrates that may be included the factor Xa reagent include fluorogenic substrate. An example fluorogenic substrate includes a nonfluorescent material that is acted upon by an enzyme to produce a fluorescent compound. In an example, a fluorogenic substrate shines or glows brightly due to fluorescence based on the amount of factor Xa activity in a test sample. The greater the amount of fluorescence, the greater the amount of factor Xa activity in the test sample. Thus, the fluorogenic substrate may enable measuring activity of the factor Xa based on the amount of fluorescence, as described below. Examples of fluorogenic substrates include, but are not limited to, Z-Gly-Gly-Arg-AMC, Technothrombin^{®}, 6-amino-1-naphthalenesulfonamide-based (ANSN) fluorogenic substrate (Mes-D-LGR-ANSN(C₂H₅)₂), and/or Calibrated Automated Thrombogram.

Examples of factor Xa inhibitors that may be detected using a factor Xa reagent described herein include direct factor Xa inhibitors. A direct factor Xa inhibitor blocks factor Xa, preventing the conversion of prothrombin to thrombin in the final common pathway of clot formation. Examples of such direct factor Xa inhibitors include, but are not limited to rivaroxaban, apixaban, edoxaban, and betrixaban.

Examples of factor Xa inhibitors that may be detected using a factor Xa reagent described herein include indirect factor Xa inhibitors. Examples of indirect factor Xa inhibitors are based on antithrombin. Antithrombin is a plasma glycoprotein that inhibits thrombin and other activated serine proteases, including factor Xa. Examples of indirect factor Xa inhibitors include, but are not limited to, one or more of low molecular weight heparin, unfractionated heparin, fondaparinux, and danaparoid.

### CHROMOGENIC AND FLUOROGENIC ASSAYS

In some implementations, detecting factor Xa inhibitors or reversal of such inhibitors in a test sample may be performed using a chromogenic or fluorogenic assay. The chromogenic assay uses colored substrates to perform detection. The fluorogenic assay uses fluorescent substrates to perform detection. In each assay, one or more curves may be generated beforehand based on known amounts of factor Xa activity as defined by fluorescence values or absorbance values and corresponding known concentrations of factor Xa inhibitors in a reaction mixture. In some implementations, one or more curves may be generated for different factor Xa inhibitors. In cases where the assays are performed on diagnostic test equipment or clinical analyzers, the curves may be stored in memory for retrieval when an assay is performed on a test sample. The curves may be used to detect factor Xa inhibitors, and the concentrations thereof, in a reaction mixture.

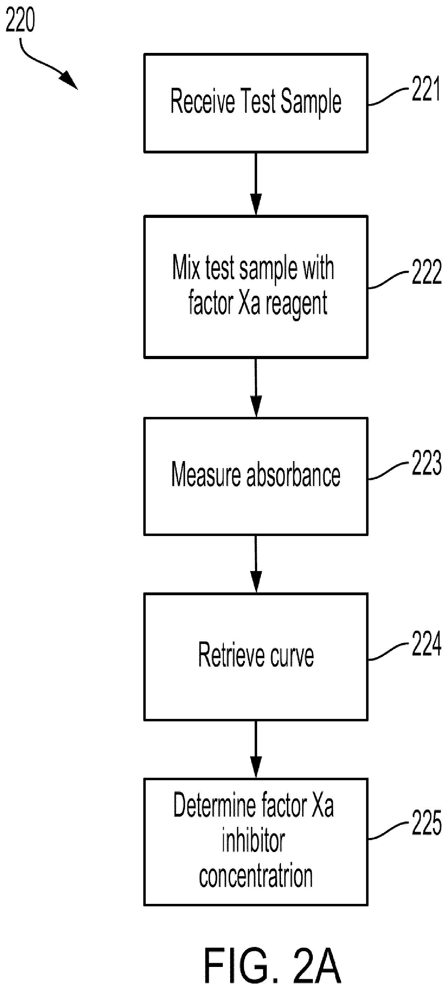

Fig. 2A illustrates an example process 220 that may be performed to detect factor Xa inhibitors in a test sample using a chromogenic assay. According to process 220, a test sample is received (221) and is mixed, combined or contacted (222) with a factor Xa reagent containing a chromogenic substrate. An absorbance of the cleaved chromogenic substrate, which represents residual factor Xa activity, in the resulting mixture is spectrophotometrically measured (223). In this example, the absorbances of a series of samples having known concentrations of factor Xa inhibitors were previously measured and used to create a curve that relates absorbance values to known factor Xa inhibitor concentrations. This curve is retrieved in operation 224. The factor Xa inhibitor concentration in the test sample is determined (225) by comparing the measured absorbance value of the mixture with the curve. That is, the absorbance of the mixture is identified on the curve and the corresponding factor Xa inhibitor concentration is obtained from the curve. If a test sample is known beforehand to contain a known concentration of factor Xa inhibitor, the result of this assay may indicate that effects of the factor Xa inhibitor have been reversed if the factor Xa activity that is determined is not commensurate with the expected effects of the known factor Xa inhibitor on the test sample.

Determining factor Xa inhibitor concentration is not limited to processes using the curve prepared in this example. In some implementations, additional methods, for example machine learning algorithms, may use the results of a chromogenic assay (e.g., absorbance and/or other parameters derived from the results) to determine the factor Xa inhibitor concentration in a test sample.

In some implementations, chromogenic assays may be performed on an ACL TOP^{®} Family hemostasis testing system provided by Werfen^{®} S.A. That system includes a fully automated system for loading and unloading cuvettes holding a mixture of test sample such as blood and a factor Xa reagent described herein. In another non-limiting example, chromogenic assays may be performed on a SPECTROstar Nano^{®} absorbance microplate reader provided by BMG LABTECH^{®} Inc. That system includes a microplate- or cuvette-based system for reading full-spectrum absorbance of a mixture of a test sample and a factor Xa reagent described herein.

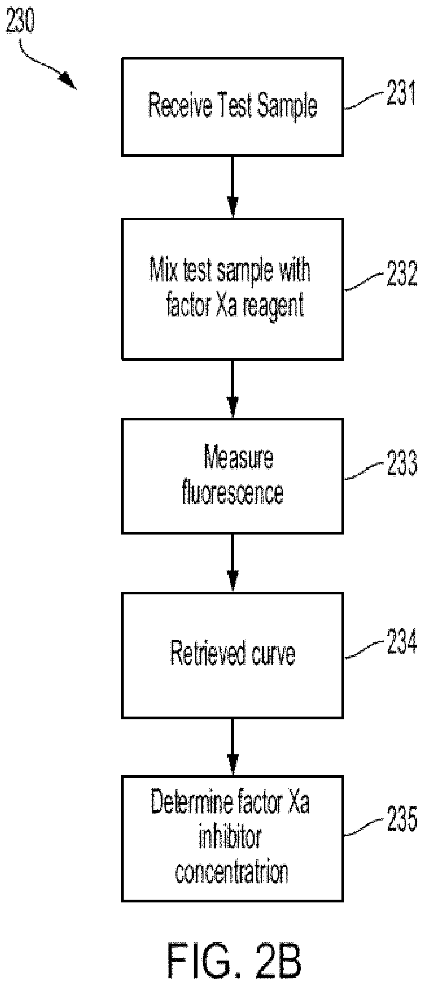

Fig. 2B illustrates an example process 230 that may be performed to detect factor Xa inhibitors in a test sample using a fluorogenic assay. The test sample is received (231) and is mixed, combined, or contacted (232) with the factor Xa reagent containing a fluorogenic substrate. The fluorescence of the cleaved fluorogenic substrate in the resulting mixture, which represents residual factor Xa activity, is measured (233). In this example, the fluorescence from a series of samples having known factor Xa inhibitor concentrations were previously measured and used to create a curve described above that relates fluorescence values to known factor Xa inhibitor concentrations. The curve is retrieved in operation 234. The factor Xa inhibitor concentration in the test sample is determined (235) by comparing the fluorescence of the mixture with the curve of the known factor Xa inhibitor concentrations. That is, the fluorescence of the mixture is identified on the curve and the corresponding factor Xa inhibitor concentrations is obtained from the curve. As was the case above, if a test sample is known beforehand to contain a known concentration of factor Xa inhibitor, the result of this assay may indicate that effects of the factor Xa inhibitor have been reversed if the factor Xa activity that is determined is not commensurate with the expected effects of the known factor Xa inhibitor on the test sample.

Determining factor Xa inhibitor concentrations is not limited to processes using the curve prepared in this example. In some implementations, additional methods, for example machine learning algorithms, use the results of a fluorogenic assay (e.g., fluorescence and/or other parameters derived from the results) to determine the factor Xa inhibitor concentration in the test sample.

In some implementations, fluorogenic assays may be performed on the PHERAstar^{®} FSX fluorescence microplate reader provided by BMG LABTECH^{®} Inc. That system includes a microplate-based system for reading high throughput, multi-mode reading of test samples and the factor Xa reagents described herein. In another non-limiting example, fluorescent assays may be performed on the Xenius XOF spectrofluorometer provided by SAFAS^{®} Ltd. That system includes a microplate- or cuvette-based system for reading *in situ*, high throughput reading of test samples and the factor Xa reagents described herein.

### ANTITHROMBIN DETECTION

In some implementations, the composition of the factor Xa reagent may include heparin such that the composition of the reagent includes factor Xa, a substrate such as a chromogenic or fluorogenic substrate, one or more of guanidine derivatives, and heparin. In some implementations, heparin may be included in the reagent at a concentration in a range of about 100 to 4000 U/L (units per liter). An aqueous buffer may be included in a liquid version of the reagent.

The factor Xa reagent containing heparin may be used to detect antithrombin in a test sample by measuring factor Xa activity. Antithrombin is a natural anticoagulant comprised of a glycoprotein that inactivates enzymes of a coagulation system. The heparin and factor Xa in the reagent form a complex with antithrombin that inhibits factor Xa activity in a test sample. The resulting residual factor Xa activity in the test sample is inversely proportional to the amount of antithrombin in the test sample. For example, greater amounts of antithrombin in a test sample will result lower amounts of factor Xa activity and lesser amounts of antithrombin in the test sample will result in greater amounts of factor Xa activity.

The systems described above may be used to perform a chromogenic or fluorogenic assay on the test sample. For example, the factor Xa activity in the test sample may be measured following contact with the factor Xa reagent containing heparin by measuring absorbance or fluorescence of the substrate in the test sample as described above. The resulting measurement may be compared to a predefined curve relating absorbance or fluorescence to known values of antithrombin in a manner similar to that described with respect to Figs. 2A and 2B. The antithrombin concentration in the test sample may thus be determined to be the value of antithrombin on the curve that corresponds to the measured absorbance or fluorescence of the substrate in the test sample.

### ALKYLATED GUANIDINES MODULATE FACTOR Xa ACTIVITY

This experiment is presented to show that alkylated guanidine derivatives modulate factor Xa activity.

In this experiment, activity of factor Xa in the presence of guanidine sulfate and its derivatives (e.g., 1-methylguanidine sulfate, 1,1-dimethylguanidine sulfate, 1,1-diethylguanidine, N-benzyl, N-methylguanidine sulfate, 1,1,3,3-tetramethylgunidine and 1,1-dimethylbiguanide HCl) was evaluated using a chromogenic assay. In the experiment, each guanidine derivative was dissolved in deionized (DI) water to a final concentration of 100mM. A number of such guanidine dilutions were prepared. Guanidine derivative levels in the solutions were tested in triplicate at 37°C as follows: 40µL (microliters) of each guanidine derivative level was incubated for 20 seconds (s) to 60s before 40µL of 6 nkat/mL factor Xa in 20mM Hepes pH 7.5 buffer was added to the reaction. After an incubation period of 150s to 180s, 100µL of 1.5mM factor Xa chromogenic substrate S-2732^{™} was added to the reaction and milli-absorbance per minute (mAbs/min) at 405nm (nanometers) was measured after a 20s delay.

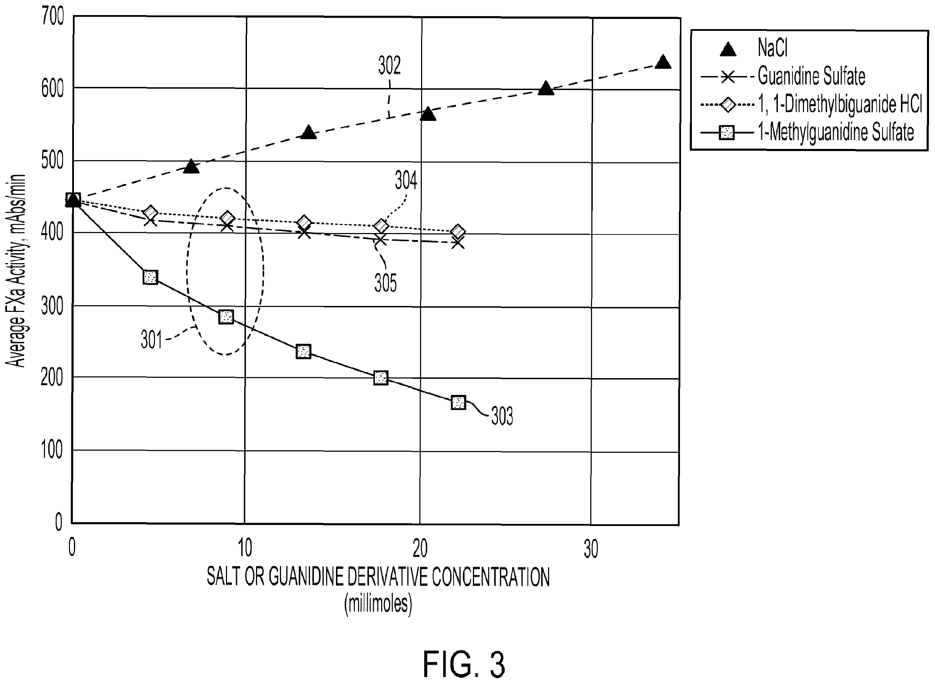

Fig. 3 shows the results of this experiment including factor Xa activity plotted against guanidine derivatives 301 or NaCl (salt) 302 concentrations in millimoles. As shown, NaCl 302 activates factor Xa activity. By contrast 1-methylated guanidine sulfate 303 modulates factor Xa dose-dependently, 1,1-dimethyl-biguanide 304, the active ingredient in metformin, and guanidine sulfate 305 also modulate factor Xa dose-dependently, although less than 1-methylated guanidine sulfate 303.

### EFFECT OF ALKYLATED GUANIDINES ON FACTOR Xa ACTIVITY

This experiment was performed and shows that guanidine and derivatives thereof modulate factor Xa activity.

In this experiment, the effects of guanidine sulfate and the guanidine derivatives: 1-methylguanidine sulfate, 1,1-dimethylguanidine sulfate, 1,1-diethylguanidine, and N-benzyl, N-methylguanidine sulfate were analyzed using a chromogenic assay. A number of dilutions were prepared in water with guanidine or a guanidine derivative of the type described herein. 40µL of each of the guanidine or guanidine derivatives were mixed for 20s to 60s with 40µL of 6nkat/mL factor Xa diluted in 20mM HEPES, pH 7.5 buffer. After an incubation period of 150s to 180s, 100µL of the factor Xa chromogenic substrate S-2732^{™} (1.5 mM) was added to each of the guanidine and factor Xa mixtures and each mixture was chromogenically measured at 405nm mAbs/min after a 20s delay.

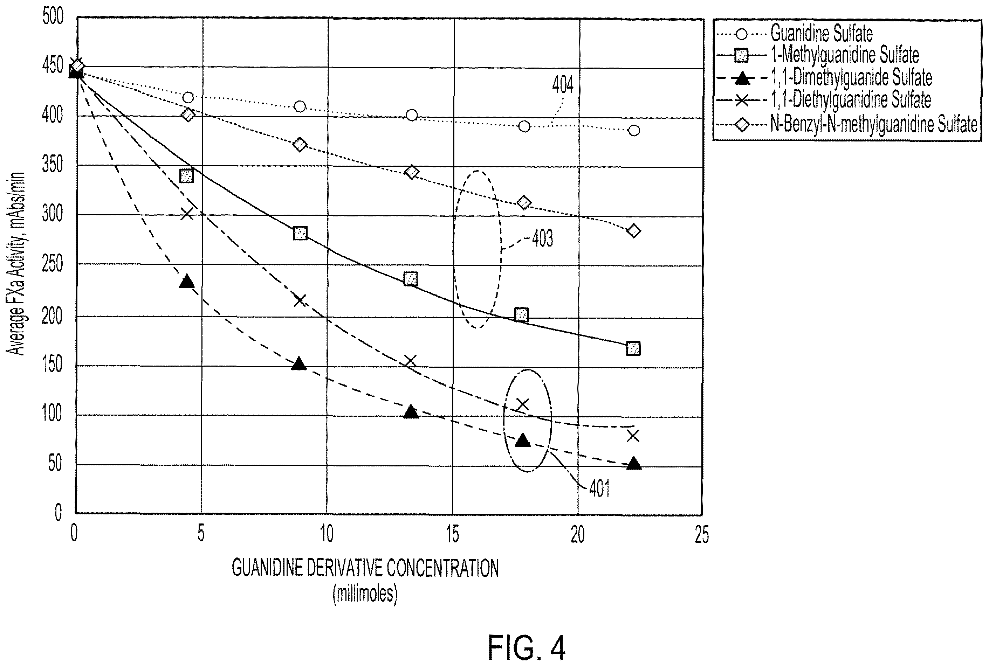

Fig. 4 shows the results of this experiment. As shown in Fig. 4, the largest extent of factor Xa inhibition was seen in mixtures containing dialkylated guanidines 401 (1,1-dimethylguanidine sulfate and 1,1-diethylguanidine sulfate). Mixtures 403 containing 1-methylguanidine sulfate and a guanidine derivative containing a benzyl group (N-benzyl N-methylguanidine sulfate) led to a reduced factor Xa inhibition as compared to the factor Xa inhibition of mixtures containing dialkylated guanidine derivatives 401. Mixtures containing guanidine sulfate 404 alone led to the least amount of reduction in factor Xa inhibition.

The results also show that the inhibition of factor Xa activity by methylated guanidine is enhanced by methylation at the second 1N position. Introducing a bulky benzyl group to the 1N position leads to less factor Xa inhibition. The inhibition can be modulated by changing the length of alkyl groups.

In some implementations different guanidine derivatives, such as two or more of the guanidine derivatives described here, can be combined in compositions to modulate factor Xa activity. In some other implementations, one or more guanidine derivatives and a factor Xa activator, such as NaCl, can be combined in compositions to modulate the factor Xa activity.

### ALKYLATED GUANIDINES ENABLE DETECTION OF FACTOR Xa INHIBITORS

This experiment was performed to show that guanidine and derivatives thereof may be included in a reagent to detect multiple types of factor Xa inhibitors.

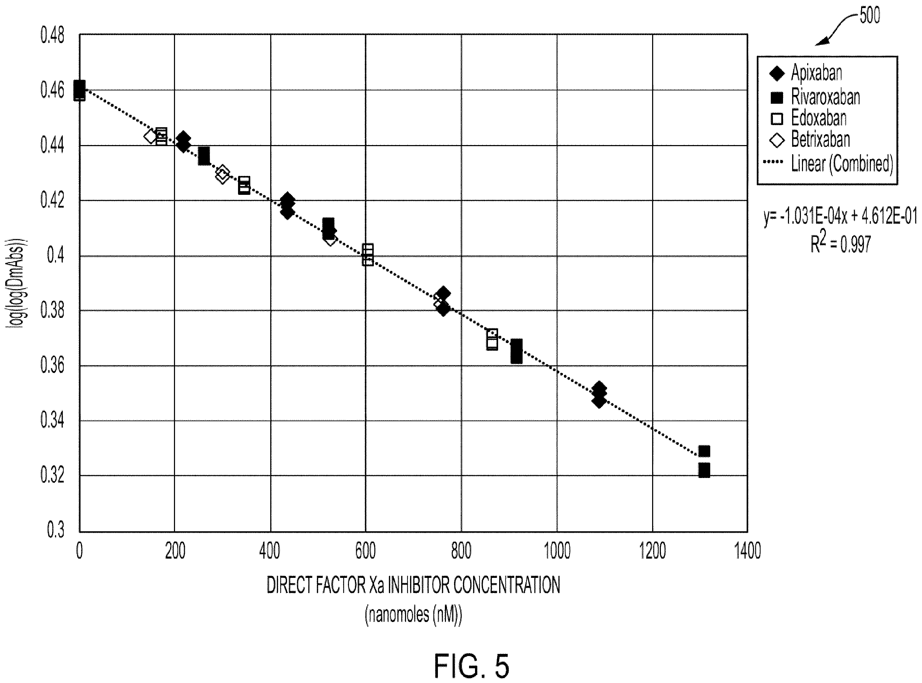

In this experiment, 100mM of dimethylguanidine was used to replace NaCl in a liquid anti-factor Xa assay for measurement of factor Xa inhibitors 500. In this example, a plasma sample, spiked with either apixaban, rivaroxaban, edoxaban or betrixaban, was assigned a concentration in nanomoles using a set of apixaban calibrators, although calibrators for the other factor Xa inhibitors may be used. A series of different amounts of each factor Xa inhibitor were added to the spiked sample and a neat plasma sample containing no factor Xa inhibitors. Each sample was tested in triplicate and a linear regression was generated based on a log(log(DmAbs)) of the reaction relative to direct factor Xa inhibitor concentrations for the four factor Xa inhibitors combined. As shown in the example plot of Fig. 5, the factor Xa reagent and calibrator combination may be used to detect different factor Xa inhibitors such as, but not limited to, apixaban, rivaroxaban, edoxaban, and betrixaban. Therefore, a calibration curve made using a first factor Xa inhibitor can serve as a universal calibration curve for quantifying different factor Xa calibration curves (e.g., a second, third, fourth, etc. factor Xa inhibitor), providing an equivalent concentration to the first factor Xa inhibitor.

### ALKYLATED GUANIDINES ALLOW A RANGE OF FACTOR Xa CONCENTRATIONS TO BE USED IN AN ASSAY

This experiment shows that guanidine and derivatives thereof enable use of a range of concentrations of factor Xa in an assay.

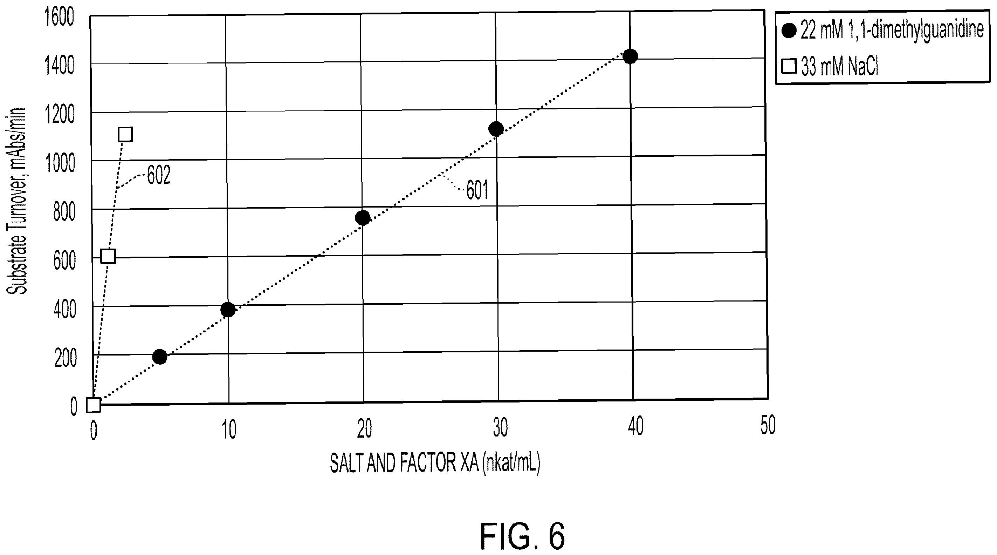

Guanidine derivatives modulate factor Xa enzymatic activity in a reaction, as explained previously, thereby slowing-down the reaction. As a result, greater amounts and/or concentrations of factor Xa can be used in a reaction when a guanidine derivative is incorporated into the reaction. In this experiment, factor Xa levels were tested in a reaction in triplicate at 37°C as follows: 40µL of factor Xa was incubated for 20s to 60s before 40µL of 100mM 1,1-dimethylguanidine or 150mM NaCl in water was added to the reaction. After an incubation period of 150s to 180s, 100µL of 1.5mM factor Xa chromogenic substrate S-2732^{™} was added to the reaction and mAbs/min at 405 nm was measured after a 20s delay. In Fig. 6, substrate turnover rates (e.g., coloring of the substrate) in the presence of 22mM 1,1-dimethylguanidine 601 were compared to substrate turnover rates in the presence of 33 mM NaCl 602. As evident from Fig. 6, greater amounts and/or concentrations of factor Xa can be added to the reaction in the presence of 1,1-dimethylguanidine to produce the same substrate turnover rates relative to NaCl. This is because the reaction has been slowed-down by the 1,1-dimethylguanidine. Greater amounts and/or concentrations of factor Xa facilitate detection of factor Xa inhibitors or reversal of such inhibitors in an assay, as noted previously.

### EFFECTS OF ALKYLATED GUANIDINES ON REACTION ACTIVITY

This experiment was performed to show the effects of a guanidine derivative on reaction activity.

In an example factor Xa inhibitor measurement setting, approximately 5% sample is mixed with a reagent and factor Xa concentration at about 2nkat/mL. A relatively low sample-to-reagent volume ratio such as 1:20 (where "1" is the proportion of sample and 20 is the proportion of reagent) may yield test results that are not clinically relevant due to dissociation of complexes in the sample or inadequate inhibition of factor Xa from the reagent. These issues may be addressed by using a factor Xa reagent of the type described herein. In some implementations, the factor Xa reagent enables test results that are clinically relevant using sample-to-reagent volume ratios that range, e.g., from about 1:10 to about 50:1 (in other words, one part sample to ten parts reagent to fifty parts sample to one part reagent).

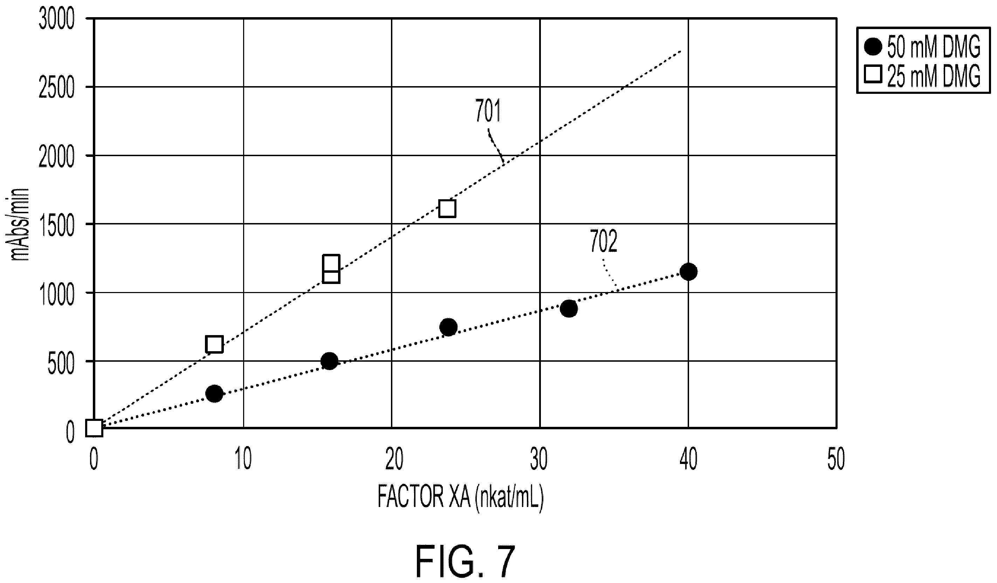

In this experiment, 180µL of a plasma sample containing dimethylguanidine (DMG) and 1.5 mM substrate S-2732^{™} were mixed with 20µL of factor Xa (a 9:1 sample to factor Xa ratio). The activity of factor Xa was determined at 37°C based on chromogenic activity of the substrate. The activity of factor Xa linearly correlates with the factor Xa concentration in the presence of dimethylguanidine. As shown in Fig. 7, 50mM of dimethylguanidine 702 slows reaction of the factor Xa in a sample greater than 25mM of dimethylguanidine 701. Thus, increasing the amount of dimethylguanidine in a sample reduces a rate of the reaction. In some implementations, dimethylguanidine can be added to a reaction at a concentration of up to about 500mM.

### EFFECTS OF ALKYLATED GUANIDINES ON ACTIVITY OF REACTION THAT INCLUDES UNFRACTIONATED HEPARIN (UFH)

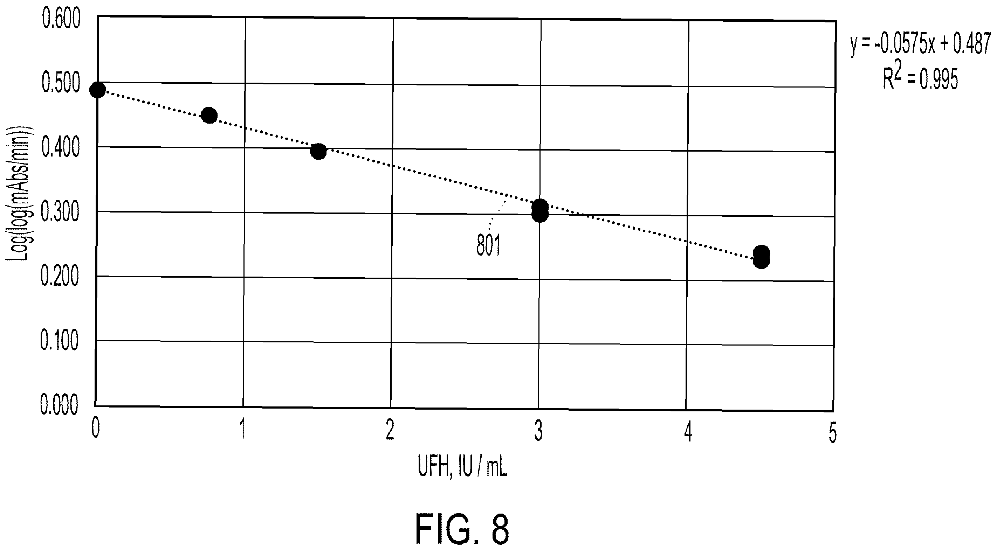

This experiment was performed to show the effects of adding a guanidine derivative to a reaction that includes unfractionated heparin (UFH). In this experiment, 180µL of a plasma sample containing 0 to 4.5 IU/mL (international units per milliliter) of UFH, 56mM dimethylguanidine, and 1.5mM substrate S-2732^{™} were mixed with 20µL of 475nkat/mL of factor Xa. In the resulting reaction, UFH and antithrombin in the plasma sample forms a ternary complex with factor Xa to inhibit factor Xa activity. Residual activity of unbound factor Xa was determined at 37°C. As shown in Fig. 8, the measured factor Xa activity on the Y axis linearly correlates 801 with UFH concentration in plasma samples in the presence of dimethylguanidine on the X axis. Accordingly, by determining factor Xa activity in a reaction, e.g., based on chromogenic activity, it is possible to use this linear correlation determine the concentration of an indirect factor Xa inhibitor, such as UFH, in a test sample.

### ANTITHROMBIN ACTIVITY

These experiments were performed to illustrate factor Xa amidolytic activity as compared to antithrombin activity in the absence and presence of a guanidine derivative which, in this example, is dimethylguanidine.

In these experiments, a plasma sample, containing 99% antithrombin activity was diluted by 15-fold with an antithrombin-deficient plasma and tested for inhibition of factor Xa activity in the presence of heparin.

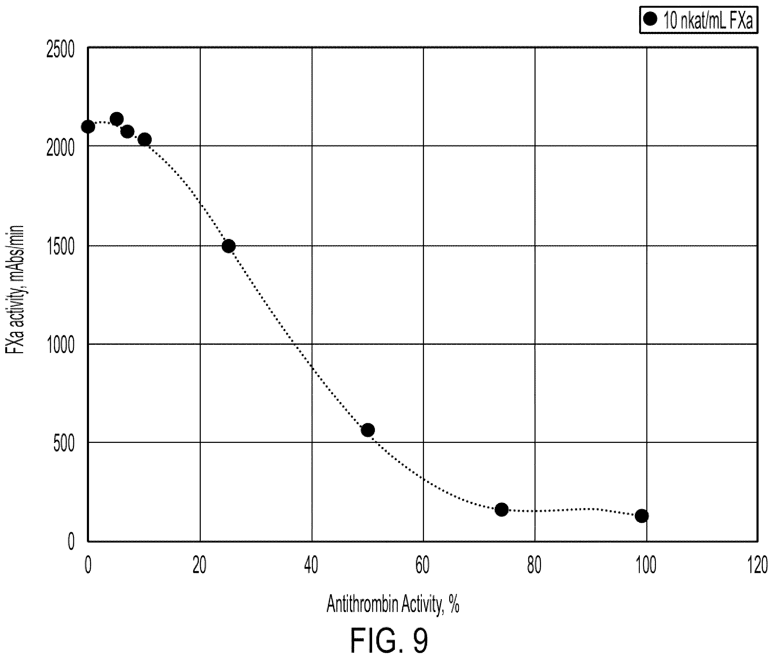

In the experiment that produced the plot of Fig. 9, 50µL of the foregoing plasma sample was mixed with 50µL of a reagent containing 10nkat/mL factor Xa and heparin, and then the residual factor Xa activity was measured by adding 50µL of chromogenic substrate S-2765^{™}. A panel of plasma samples containing varied levels of antithrombin was prepared by diluting the plasma sample with physiological saline and tested. As shown in Fig. 9, lower levels of antithrombin activity produce greater levels of factor Xa activity and greater levels of antithrombin activity produce lower amounts of factor Xa activity.

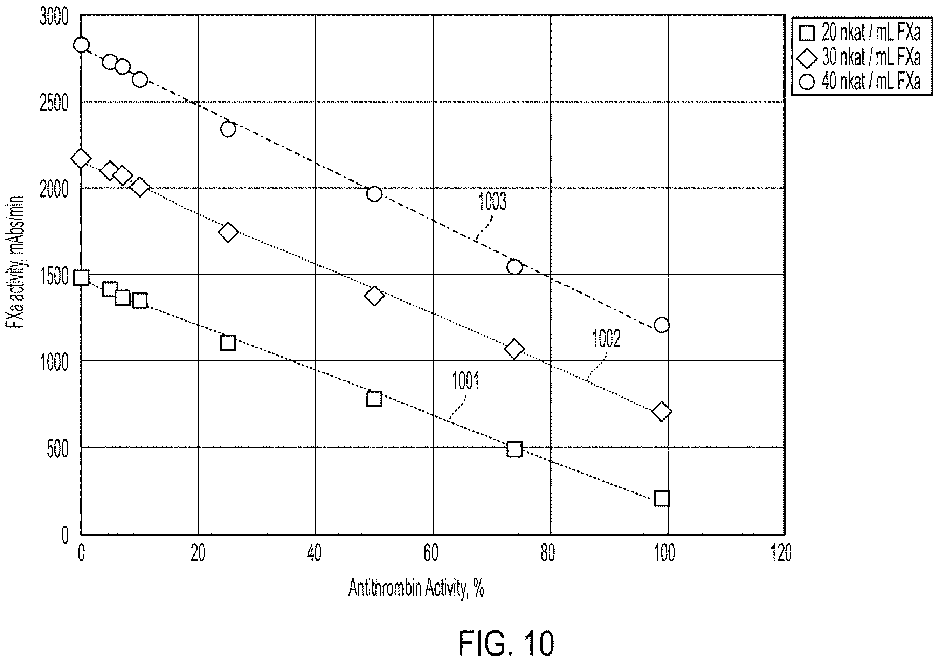

In the experiment that produced the plots of Fig. 10, 50µL of the foregoing plasma sample was mixed with 50µL of a reagent containing 20 nkat/mL1001 factor Xa and heparin, with 50µL of a reagent containing 30 nkat/mL1002 factor Xa and heparin, and with 50µL of a reagent containing 40 nkat/mL1003 factor Xa and heparin. In each case, the residual factor Xa activity was measured by adding 50µL of factor Xa chromogenic substrate S-2765^{™} containing 60mM dimethylguanidine. As shown in Fig. 10. when modulated by dimethylguanidine, factor Xa activity responded linearly over a range of antithrombin activity. By using plots like those of Fig. 10, knowing the concentration of factor Xa in the reagent and the resulting measured factor Xa activity in a test sample, it is possible to determine the antithrombin activity, and thus the amount of antithrombin, in the test sample.

Assays that use the factor Xa reagents described herein can be performed manually or automatically. For example, the reagents may be manually or automatically activated, e.g., using robotics manipulators or flow-through fluidic devices to mix the reagents with test samples. Software applications may be used to analyze the test results to provide or calculate parameters. The software applications may be used to create additional information that may be used, for example, to inform clinical actions.

The use of the factor Xa reagents is not limited by the examples described in this disclosure, and the applicable assays are not limited by any particular system for implementation. Systems can be for laboratory use, such as a central laboratory for clinical testing, and can be for point-of-care use, such as for first responders, or in an operating room.

The factor Xa reagents may be packaged and stored in bulk volumes, single use volumes, multiple use volumes, or test volumes. The factor Xa reagents may be packaged and stored in any form, including forms for kits or cartridges. For example, a cartridge storing the factor Xa reagent in liquid or dry form or components thereof such as factor Xa, a substrate, and one or more guanidine derivatives in liquid and/or dry form. The cartridge may be configured to be inserted into an instrument and for measurement of factor Xa inhibitors. The cartridge, coupled or inserted into the instrument, may also be configured to receive a test sample, such as whole blood, to mix the factor Xa reagent with the test sample, and to move the resulting mixture to a position for measurement of the absorbance or fluorescence of the mixture. The cartridge can be for single or multiple use and can be disposable once the reagents therein are spent. The cartridge can also include a reference or calibration reagent to confirm the cartridge and or instrument is operating within acceptable parameters or create a calibration curve. In some implementations, the reference or calibration reagent is a direct or an indirect factor Xa inhibitor as described herein.

For example, the kit may contain the factor Xa reagent in liquid or dry form, or components thereof such as factor Xa, a substrate, and one or more guanidine derivatives in liquid and/or dry form. These can be packaged in containers for multiple use, or containers for single use. The kit may also include an aqueous buffer, examples of which are described herein. The kit can also include a calibration or reference reagent, for example, to confirm the kit and or instrument is operating within acceptable parameters or create a calibration curve. In some implementations, the reference or calibration reagent is a direct or an indirect factor Xa inhibitor as described herein.

The diagnostic test instruments, clinical analyzers, and/or assays described herein may be controlled using computing systems or any other appropriate computing device having one or more processing devices, such as one or more microprocessors, one or more microcontrollers, or programmable logic such as one or more field-programmable gate arrays (FGPAs) or one or more application-specific integrated circuits (ASICs). The diagnostic test instruments and clinical analyzers described herein may execute one or more computer program products, e.g., one or more computer programs tangibly embodied in one or more information carriers, such as one or more non-transitory machine-readable media, to control the assays and to implement all or part of the processes described herein.

Elements of different implementations described herein may be combined to form other implementations not specifically set forth above. Elements may be left out of the structures described herein without adversely affecting their operation. Furthermore, various separate elements may be combined into one or more individual elements to perform the functions described herein.

## Claims

1. A composition comprising:
factor Xa;
a substrate; and
one or more of guanidine derivatives,
wherein the one or more guanidine derivatives comprise alkylated guanidine having a chemical structure represented by formula: 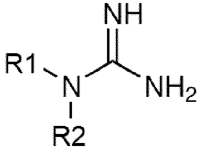
wherein R1 is H, methyl, ethyl, or benzyl;
wherein R2 is H, methyl, ethyl, or benzyl; and
wherein N is nitrogen and H is hydrogen.

2. The composition of claim 1, further comprising:
heparin.

3. The composition of claim 1, wherein the one or more guanidine derivatives comprise 1-methylguanidine, 1,1-dimethylguanidine, 1,1-diethylguanidine, or N-benzyl-N-methylguanidine, where N indicates a substituent on nitrogen.

4. The composition of claim 1, wherein the one or more guanidine derivatives comprise a mono-alkylated guanidine derivative, or wherein the one or more guanidine derivatives comprise a dialkylated guanidine derivative.

5. The composition of claim 1, wherein the one or more guanidine derivatives comprise:
a mono-alkylated guanidine comprising an alkyl group having a length that is different from a length of an alkyl group of 1-methylguanidine; or
a dialkylated guanidine comprising an alkyl group having a length that is different from a length of an alkyl group of 1,1-dimethylguanidine or 1,1-diethylguanidine; or
wherein the one or more guanidine derivatives comprise a sulfate salt.

6. The composition of claim 1, wherein the substrate comprises a chromogenic substrate.

7. An apparatus containing the composition of claim 1.

8. The apparatus of claim 7, wherein the apparatus is a cartridge, or wherein the apparatus is a kit.

9. A method for detecting an anticoagulant in a sample, the method comprising:
combining or contacting the sample with the composition of any one of claims 1 to 6; and
measuring activity of the factor Xa based on a state of the substrate, where a presence of the anticoagulant is based on the activity of the factor Xa.

10. The method of claim 9, wherein the anticoagulant comprises a factor Xa inhibitor comprising a direct factor Xa inhibitor, preferably one or more of rivaroxaban, apixaban, edoxaban, or betrixaban.

11. The method of claim 9, wherein the anticoagulant comprises a factor Xa inhibitor comprising an indirect factor Xa inhibitor based on antithrombin, preferably one or more of low molecular weight heparin, unfractionated heparin, fondaparinux, or danaparoid.

12. The method of claim 9, wherein the state of the substrate comprises a color change in the substrate.

13. The method of claim 9, wherein the composition modulates the activity of the factor Xa in the sample, where modulation comprises reducing a rate of coagulation of the sample caused by factor Xa.

14. The method of claim 9, wherein the composition further comprises heparin; and wherein the anticoagulant is antithrombin.
